**Europäisches Patentamt**

(19) **European Patent Office**

**Office européen des brevets**

(11) Publication number: **0 020 496**
**B2**

(12) **NEW EUROPEAN PATENT SPECIFICATION**

(45) Date of publication of the new patent specification:
27.01.88

(21) Application number: 79901350.3

(22) Date of filing: 26.09.79

(86) International application number:
PCT/US 79/00787

(87) International publication number:
WO 80/00659 (17.04.80 Gazette 80/8)

(51) Int. Cl.⁴: **A 61 K 9/32**, A 61 K 9/36,
C 08 L 1/32, C 08 L 1/12,
C 08 L 1/26

(54) FOODS AND PHARMACEUTICAL COATING COMPOSITION, METHOD OF PREPARATION AND PRODUCTS SO COATED.

(30) Priority: 02.10.78 US 947948

(43) Date of publication of application:
07.01.81 Bulletin 81/1

(45) Publication of the grant of the patent:
11.04.84 Bulletin 84/15

(45) Mention of the opposition decision:
27.01.88 Bulletin 88/4

(84) Designated Contracting States:
CH DE FR GB LU NL SE

(73) Proprietor: **PURDUE RESEARCH FOUNDATION**, Hovde
Hall Purdue University, West Lafayette
Indiana 47907 (US)

(72) Inventor: **BANKER, Gilbert Stephen**, 1210 Western Drive,
West Lafayette, IN 47906 (US)

(74) Representative: **Warden, John Christopher, R.G.C.
Jenkins & Co. 12-15, Fetter Lane, London EC4A 1PL
(GB)**

(56) References cited:
EP - A - 0 113 443
BE - A - 830 570
FR - A - 1 595 925
FR - A - 2 145 642
FR - A - 2 274 281
FR - A - 2 364 660
FR - A - 2 378 832
US - A - 2 722 528
US - A - 2 843 583
US - A - 3 196 827
US - A - 3 522 070
US - A - 3 935 326
US - A - 4 060 598

KIRK-OTHMER, "Encyclopedia of Polymer Science and
Technology", 1965, Vol. 2, p. 387 N.W. DESROSIER,

(56) References cited: (continuation)
"The Technology of Food Preservation", 4th Ed., 1978,
Avi Publishing Co., p. 393-396
DERWENT, Pub. Ltd. 78752 W/48
DERWENT Pub. Ltd. 15108 W/09
UMWELT und CHEMIE von A-Z, 3. Ed., 1978, p. 42
Handbook of Chemistry and Physics, 46th Ed., The
Chemical Rubber Co., Cleveland, Ohio, USA, p. F-56
The Condensed Chemical Dictionary, 8th Ed., Van
Nostrand Reinhold Co. p. 228
DTV-Lexikon der Physik, Bd. 6, Lie-OZ, p. 151
RÖMPPS CHEMIE-LEXIKON, 8th Ed., Franckh'sche
Verlagshandlung, Stuttgart, p. 132

The file contains technical information submitted after
the application was filed and not included in this
specification

ACTORUM AG

## Description

The invention relates generally to food and pharmaceutical coating compositions, a process for producing the coating compositions, and more particularly to a pharmaceutical tablet, capsule, bead or particulate coating in the form of an aqueous latex of water insoluble polymeric compositions, and to solid food or pharmaceutical products coated with such materials.

The use of various coatings to seal pharmaceutical (including vitamins and food supplements) dosages and food products has been known for some time. Many ends have been served by such coatings. For instance, tablet coatings provide a more attractive appearing dosage, protect the dosage from attack, such as by moisture during storage, provide a more acceptable feel, and often taste, to the user of the dosage, and, with selected compounding, provides either gastric and/or enteric dissolution at varying rates to determine the time and rate at which the dosage is released.

Early coatings include natural and synthetic polymers such as cellulose acetate phthalate and shellac to, as the primary purpose, delay release of the dosage thus providing enteric dosage. Sugar coating of the dosage is a classic example of an early pharmaceutical tablet coating affording gastric release, but has the drawback of being a very involved process requiring highly skilled operators. As a result of the various advantages and disadvantages of known coatings for pharmaceutical dosages, film coatings applied to the dosages from organosols (that is), from solutions of plasticized polymers and organic solvents, have become the most utilized procedure. Film coatings from organosols provide rapid, simple, effective coating of the dosage and control over the dosage size, colating disintegration and dissolution rates, and generally results in an attractive and desirable dosage coating. Generally, the organic solvents curtailed bacterial and mold growth.

A number of disadvantages have also been attendant to the organosol coatings of pharmaceutical dosages. Often the vapors of the organic solvents are toxic or flammable thereby providing a hazard to the operators – though the solvents may be fully or substantially driven from the tablet coating providing no hazard to the user of the pharmaceutical dosage. Also, organic solvents present an environmental hazard and, accordingly, require recovery systems which are both expensive and complicated. As a result, in an effort to reduce the amount of solvents employed in film coating, fillers and extenders are often added to the film coating formulations. While this results in an increased solids loading of the coating dispersion, the additives and extenders often compromise the performance of the tablet coating in terms of mechanical properties and permeability. Except in high volume operations, the cost of recovery equipment for the organic solvents often proves prohibitive despite the high cost of the solvents recovered by such systems for recycling. Independent of the fundamental economics of such recovery systems, the demands of environmentally responsible manufacturing often mandate such solvent recovery systems with resulting adverse impacts on the economics of many film coating operations.

Because of these difficulties, certain manufacturers utilised the technique of emulsion polymerisation to form tablet coatings (see e.g. US–A 3 935 326).

Water, of course, is an excellent solvent or vehicle for many practical purposes in that it is non-flammable and thus provides no explosion hazard, is low cost, is substantially free of toxic effects and innocuous to the environment. Unfortunately, the use of aqueous solutions of polymers for film coating to provide dosage coatings is quite limited due to the verly low solids contents, i.e., on the order of 7% to 15% of known aqueous solutions. As the concentration of solids rises, the viscosity of the solution rapidly approaches an inoperable condition. Similarly, an increase in the molecular weight of the polymer in an aqueous solution rapidly causes and unworkably high viscosity. Thus, relatively low molecular weight polymers and low concentrations are normally required. These characteristics result in a need for a plurality of layers of polymer to be built up in order to obtain a coating of adequate thickness for surface protection. Multiple coatings involve a long processing time which is complicated further by the slow evaporation of water and large amounts of water which must be removed from each coating. Further, many drugs are sensitive to water when exposed to high concentrations for extended periods of time and, accordingly, require initial sealing with water barriers, such as the shellac coating, prior to coating with the conventional aqueous film coating.

Another serious drawback with existing water-based coating systems is the tendency for bacteria and molds to multiply and grow in aqueous media, either solvent or vehicle. Thus care in storage and attention to shelflife are of critical importance in avoiding septic conditions in known aqueous based coating systems.

Still another problem with the low solids aqueous film coating techniques now known is the relatively poor film obtained. Few water soluble materials provide the desired film coatings properties. The more desirable film obtainable with the use of longer chains, higher molecular weight polymers cannot be employed in the conventional aqueous coating methods.

It is known for the purpose of making certain paints or varnishes (see e.g. US-A 2 722 528, 2 843 583 and FR-A 2 378 832) to use a technique in which a polymer is first dissolved in a volatile, water immiscible solvent, the solution is then dispersed in water and then the solvent is removed by distillation.

By adopting this technique the applicants have found it possible to produce stable latices with a high solids loading, resistant to bacteria and mold growth. However, only by the use of further modifications, as will become apparent, has it been possible to produce latices having, not only these properties but good tablet coating qualities.

In accordance with the invention there is provided a process for forming aqueous coatings on food or pharmaceutical dosage forms characterised by:

dissolving at least one substantially water insoluble polymer selected from ethyl cellulose, cellulose acetate phthalate, hydroxy propyl methyl cellulose phthalate and polyvinyl acetate phthalate in a volatile water immiscible organic solvent for the polymer;

forming a fine dispersion of the water insoluble polymer solution in a continuous aqueous phase by agitation of the water and the polymer solution, optionally in the presence of an emulsifying agent and/or an emulsion stabilizer;

forming solid particles of the water insoluble polymer in the water phase having an average particle size substantially between 0.1 and 20 μm by distillation of the organic solvent from the dispersion; adding a water soluble polymer and a plasticiser and optionally an additive to enhance the properties of the dispersion and/or improve the appearance of the coating, so as to obtain a latex having solids content from 16.67% to 60% by weight;

forming a film coating on a food or pharmaceutical dosage form by applying the latex to the dosage form and removing the water therefrom.

The invention also includes as an aspect a food or pharmaceutical dosage form coated with the dried residue of a latex which is an aqueous dispersion of at least one water insoluble polymer selected from ethyl cellulose, cellulose acetate phthalate, hydroxy propyl methyl cellulose phthalate and polyvinyl acetate phthalate having an average particle size between 0.1 and 20 μm, the solids content of the dispersion comprising from 16.7% to 60% by weight of the dispersion and containing an annealing agent in the form of a watersoluble polymer together with a plasticiser and optionally an emulsifier, or an additive to enhance the properties of the dispersion or improve the quality, performance or appearance of the product coating.

Product coating may be facilitated by adding selected additives to the latex and applying such dispersion having high solids loading to pharmaceutical dosages by conventional means in a coating pan or by more specialised procedures, i.e., immersion coating, fluidized beds, etc. US-A 3 896 762 issued July 29, 1975 discusses a workable coating apparatus.

Many water immiscible solvent systems are appropriate for use in the present invention. Primarily, the solvents or solvent systems must have substantial solvent effect upon the polymers employed, and preferably be quite volatile relative to water in order to leave little residue in the final latex or, alternatively, have permissible residue limits for use in foodstuffs. Typical solvents include chlorinated solvents such as ethylene dichloride, tetrachloroethylene, chloroform, methylene chloride and similar solvents, aliphatic, alicyclic or aromatic hydrocarbons such as hexane and cyclohexene, esters such as ethyl acetate, higher alcohols, ethers, and combinations thereof, or combinations of such solvents with polar water miscible solvents such as acetone or lower alcohols in ratios which produce an overall mixed solvent which is water immiscible. Isopropanol and methylene chloride, ethylene dichloride or chloroform, for instance, constitute an excellent water immiscible solvent system for hydroxypropyl methyl cellulose phthalate. Spice oleoresins are examples of solvents having permissible residue limits in foodstuffs. Numerous other solvent systems will be apparent to those skilled in the art.

It is often desirable to include surfactants or emulsifiers to assist in producing a stable emulsion. The emulsifiers may be anionic such as sodium lauryl sulfate (USP), cationic such as the quaternary ammonium halides (such as cetyl pyridinium chloride) or nonionic, such as linear fatty alcohol ethoxylates (exemplified by Alkasurf (trade mark) LA3, LA7, LA9 or LA12 available from Alkanol Chemical Ltd. of Mississauga, Ont., Canada) or the polyoxyethylene condensation products (exemplified by Spans (trade mark) and Tweens (trade mark) or polyoxyethylene-polypropylene glycol as Pluronic F68 (trade mark), available from Wyandotte Chemicals Corp., Wyandotte, Michigan, U.S.A.). Other agents including materials such as polyglycerol esters of fatty acids, polyoxethylene sorbitan monolaurate, polyoxethylene sorbitan tristearate, polyoxethylene sorbitan monostearate, polyoxyethylene sorbitan monoleate, propylene glycol mono and diesters of fats and fatty acids, sodium lauryl sulfate and sorbitan monostearate are useful to serve such functions. Generally, the emulsions and latices of the instant invention can be formed without surfactants or emulsifiers, but in many instances, finer particle size are attained with such additives. The particularly above-listed biodegradable polymers form emulsions and resulting latices without benefit of additives.

Various other additives, such as cetyl alcohol, beeswax (yellow, bleached or white and white natural), candelilla wax, carnauba wax, cocoa butter, fatty acids such as those in the food additives, mono, di and tri glycerides (including glycerol monostearate, monooleate, etc. and self-emulsifying glyceryl monostearate), glycerol-lacto stearate, oleate or palmitate (other self-emulsifying waxes), glyceryl-lacto esters of fatty acids (also self-emulsifying) lauric acid, lauryl alcohol, linoleic acid, octyl alcohol and acetate, and paraffin may be advantageously included to enhance the properties of the emulsion. A number of additives such as carnauba wax and chlorowax improve the appearance of the tablet coating. These, of course, can be added to the latex as polishing agents.

The resulting latex includes between 16.67 percent to 60 percent solids, optionally 20 to 50 percent, or more, solids with low, workable viscosities and non-Newtonian dilatant flow. Concentration of the water insoluble polymer in the organic solvent generally is in the range of 10 percent to 50 percent by weight, but higher concentrations are sometimes workable and desirable, provided unduly high viscosities are not produced. Surfactants/emulsifier concentrations in the emulsions may of course be zero in some instances, but when included are within the general range of 0.1 percent to 10 percent by weight, and preferably 0.5 to 2 percent. Other additives including pharmaceutically active ingredients may be included in amounts between O percent and 20 percent by weight of the emulsion, but usually between 0 percent and 10 percent by weight of the emulsion.

The resulting latex of water insoluble polymers does not in itself usually yield the desired dosage coating composition. Annealing agents, i.e. water soluble polymers such as hydroxypropyl cellulose, hydroxypropyl methylcellulose, methyl cellulose, methyl ethyl cellulose, polyvinylpyrrolidone and/or sodium carboxymethylcellulose are necessary. Gums, including official gums, such as pectin acacia-USP, tragacanth-USP, as well as non-official gums such as karaya, locust bean, chondrus, and alginic acid derivatives, etc. may also be employed as annealing agents, though generally the aforementioned acid derivatives, etc. may also be employed as annealing agents, though generally the aforementioned annealing agents are prefered. Other materials of possibly desirable application, though believed to be less desirable than those first listed, include high molecular weight carboxyvinyl polymer such as Carbopol (trade mark) 934 available from B.F. Goodrich Company, provided it is employed in amounts not above 2 or 3% concentration and is neutralized with a sodium hydroxide to be solubilized as a sodium salt. Hydroxyethyl cellulose, available as Cellosize (trade mark) from Union Carbide Company, sodium alginate, propylene glycol estr of alginic acid, available as Kelgin (trade mark), and Kelcoloid respectively from the Delco Chemical Company, polyvinyl alcohol. Also, as an example of a low molecular weight annealing agent which is not polymeric, dioctyl sodium sulfosuccinate-USP, may, in some instances, be employed. In general, annealing agents are recognized by those skilled in the art for the properties of being water soluble, suitable for ingestion, and compatible with the basic latex. Plasticizers are also necessary to provide workable coatings. Typical plasticizers include propylene glycol, glycerin, glyceryl triacetate, polyethylene glycol, triethyl citrate, tributyl citrate, diethyl phthalate and dibutyl phthalate.

Therefore, an advantage of this invention is it provides aqueous dispersions for tablet coatings utilizing high molecular weight, water insoluble polymers heretofore applicable primarily by means of organosols.

It is a further advantage of this invention to provide more economical dosage coatings utilizing water as a dispersing medium.

It is another advantage of the present invention to provide environmentally acceptable dosage coatings.

It is still another advantage of the present invention to provide aqueous vehicle dosage coating compositions having resistance to the growth of bacteria, molds and other such objectionable matter therin.

These and other advantages of the instant invention will become evident upon further consideration of the specification and the appended claims.

According to the instant invention, high solids loading latices suitable for formulating dosage coating compositions are formed by dissolving one or more relatively high molecular weight, water insoluble polymers, in a water immiscible organic solvent or solvent system. The thus formed organic solvent solution is emulsified to form an aqueous emulsion having a continuous water phase. Generally, emulsification is accomplished by initial mixing by physical agitation and then fine dispersion by sonication and/or homogenization to form the emulsion. Optionally, additives, surfactants and emulsifying agents may be added to aid in establishing and stabilizing the emulsion, but such agents tend to be of relatively low volatility. Thus, in many instances, such additives must be acceptable in the tablet coating ultimately formed. Many acceptable agents for these purposes are known. Sufficiently stable emulsions are generally formed by subjecting the organic solvent solution and water to comminuting forces by means of devices such as colloid mills, ultrasonic vibrators, homogenizers, etc. These are well known in the art of emulsification. Preferably, the emulsion is subjected to such treatment promptly upon formation of the crude emulsion.

It is not absolutely necessary that the emulsion be stable for extended periods since it is desirable that the organic solvent be promptly stripped from the emulsion. This, in the case of marginally stable emulsions, can be carried out promptly. While it is preferred that a vacuum distillation process be employed, particularly in the instance of unstable emulsions, steam distillation, either with or without vacuum, may be employed to remove the more volatile organic solvents from the emulsion.

As the organic solvent is removed, the polymer initially dissolved therein forms discrete solid particles. These particles generally are on the order of 0.1 to 20 $\mu$m average particle size, and generally below 10 $\mu$m. Alos, the particles generally are of consistent size as a result of the homogeneous nature of the emulsion from which the particles are formed, though minor amounts of particles up to 15 or 20 $\mu$m may be present.

The essentially submicron size of the polymeric particles in the remaining aqueous phase gives rise to a latex having on the order of 16.67 percent to 60 percent solids and preferably 20 to 50 percent and most preferably 25 to 35 percent solids loading with low viscosities and non-Newtonian dilatant flow.

To provide suitable coating characteristics, a plasticizer such as triacetin, propylene glycol, glycerine, triethyl citrate, diethyl phthalate, tributyl citrate or dibutyl phthalate and, as an annealing agent, a water soluble polymer are incorporated.

Pigments, release agents, and polishing agents may also be included to vary the properties, i.e., gastric release, enteric release, rapid dissolution and appearance of the coating.

The various advantages and properties of the dosage coatings in accord with the latex forming process above described will be apparent from the following examples. Examples 1 to 7 illustrate stages in the development of the invention, but are not part of the invention, which is fully illustrated in Examples 8 and 9.

Example 1

A basic latex was formed by dissolving 60 grams of ethyl cellulose having a degree of substitution of between 2.44 and 2.58 and an ethoxyl content of 48 to 49.5 percent in 240 grams of an 85 to 15 ratio of benzene ethanol solvent. The solution was allowed then to stand overnight and was then filtered through surgical gauze. 3.3 grams of n-decane was then added.

The resulting solution of the water insoluble ehtyl cellulose in the water immiscible solvent system was added drop by drop at 55°C to an aqueous solution of 4 grams of sodium laurylsulfate in 600 grams of water. A reflux condenser was attached to the flask in which the crude emulsion was formed and a marine propeller type agitator was employed to mix the solutions. The resulting emulsion was promptly submitted to ultrasonic agitation at the rate of 120 watts for 5 minutes and passed through a two stage laboratory homogeniser at 41369 kPa (6000 psi). Thus a direct emulsification of the ethyl cellulose and water immiscible solvents was produced. Thereafter, the organic solvent was removed by vacuum distillation in a rotary evaporator at 84.7 kPa (25 inches of mercury) and 45°C after adding 0.5 grams of a silicone antifoam. As the organic solvent was removed, a stable pseudolatex of essentially submicron particles of ethylcellulose was formed in the aqueous vehicle.

Example 2

Sixty grams of ethylcellulose was dissolved in 440 grams of ethylene dichloride, filtered through surgical gauze and 13 grams of cetyl alcohol was then added to the solution. The resulting solution of a water insoluble ethyl cellulose polymer in a water immiscible organic solvent was placed in a 2000 ml, 3 neck flash equipped with a reflex condenser and a marine propeller type agitator. Four grams of sodium lauryl sulfate was added to 600 grams of water and the thus formed aqueous solution was added drop by drop to the organic solution through a separatory funnel to form a continuous aqueous phase emulsion by inverse emulsification. The thus formed crude emulsion was subjected to ultrasonic agitation and homogenization as described in Example 1, and the organic solvent was similarly removed to form a latex of micron or submicron size of ethyl cellulose particles in an aqueous vehicle.

Example 3

One hundred grams of cellulose acetate phthalate was added to 700 grams of an 80 to 20 ratio of ethyl acetate isopropanol solvent. The solution was filtered and 14 grams of cetyl alcohol added. Thereafter, a solution of six grams of sodium lauryl sulfate in 1000 grams of water was added to form an emulsion as set forth in Example 2, and a latex was formed by removing the organic solvent in the manner set forth in Example 2. The resulting latex was stable and of micron or submicron particle size.

Example 4

A solution of 10 grams of polymethyl vinyl ether maleic anhydride copolymer (Gantrez (trade mark) AN 169 available from GAF Chemical Company) was added to 100 grams of an ethyl acetate solvent. A latex of micron or submicron sized particles of the copolymer was formed in conjunction with a solution of 1.5 grams of sodium lauryl sulfate in 300 grams of water by the emulsification process and removal of the organic solvents as described in Example 2.

Example 5

Ten grams of a polymer of 90 percent L-Lactic Acid and 10 percent glycolic acid having a molecular weight of 15,000 was added to 90 grams of chloroform. A stable, micron or submicron latex of the polymer was formed in conjunction with the solution of 1 gram of fatty alcohol ethoxylate (Alkasurf (trade mark) LA 3) and 200 grams of water in the manner described with reference to the emulsification and separation described in Example 2.

Example 6

Ten grams of L-Polyactic acid having a molecular weight of 15,000 was added to 90 grams of chloroform. A stable, micron or submicron latex of the polymer was formed in conjunction with an aqueous solution identical to that utilized in Example 5 by utilizing the emulsification and organic solvent removal step described in Example 2.

All of the latices produced as described at Examples 1 through 6 were stable, micron or submicron sized latices of water insoluble polymers in aqueous vehicles. Formulation of other latices utilizing water insoluble polymers which do not form latices by more conventional emulsification of colloid forming methods can be produced by methods essentially similar to those described above. Of course, differing polymers may require appropriate organic solvent systems, and such solvent systems would be water immiscible, and may require the addition of emulsifying agents and other additives. However, the nature of such solvents and additives are well recognized in the art and may be identified without undue experimentation.

However, though the latices as produced in Examples 1 through 6 have the properties of stable, high solids loading in an aqueous vehicle and are resistant to bacterial and mold growth, such latices are not necessarily appropriate for producing tablet coatings. The suspended polymers are, of course, water insoluble and thus not readily dissolvable in gastric liquids, though this quality can be put to advantage for enteric release in many instances. Further, such latices generally form marginal films, and in many cases are not film forming at all. When film is formed, often the films are not of sufficient physical integrity or attractiveness to function as a tablet coating.

To produce a dosage coating as is the object of the present invention, plasticizers, annealing agents, in the form of a water soluble polymer which may be incorporated into the water phase of the latex, and, if desired, pigments and other additives may be added to the latices. Such other additives, which include polishing agents, release agents, etc. may be incorporated at various points in the formulation process, i.e., prior to emulsification of after formulation of the latex.

Example 7

Latices formed as set forth in Example 1 were placed in petri dishes and the solvents evaporated, at 30°C, both without and with plasticizers in varying amounts to form films. The plasticizers added were U.S.P. Grade propylene glycol, glycerin, glyceryl triacetate (triacetin), and polyethylene glycol. The results obtained are shown in Table I.

TABLE I

| Plasticizer | % Plasticizer on polymer weight | % Total polymers | Film characteristics |
|---|---|---|---|
| None | 0 | 44 | Hard, brittle, glass like and transparent. Discontinuous. |
| Polyethylene glycol | 67 | 22.3 | Discontinuous, hard and brittle. |
| Propylene glycol | 40<br>60<br>80 | 22.3 | Hard divided into small pieces. Discontinuous. |
| Glycerin | 55 | 25.5 | Translucent, soft and discontinuous. |
| Glycerin | 67.5 | 24.6 | Translucent, soft. Continuous. The film has a waxy texture. |
| Triacetin | 49 | 25.3 | Transparent, Continuous. |

While the film forming properties are improved by the addition of plasticizer, and particularly when using triacetin, still transparent and soft, waxy, fims are not ideal tablet or other dosage coatings.

Example 8

In addition to plasticizers, water soluble polymeric materials were also slowly added with gentle mixing and in various amounts to latices essentially as prepared in Example 1. The results are shown in Table II.

TABLE II

| Polymers ratio* | Water soluble polymer | Plasticizer | % Total polymers | % Plasticizer based on total polymer weight | Total solids loading % | Film characteristics |
|---|---|---|---|---|---|---|
| 1:1 | Methylcellulose 50 mPa. s | Propylene glycol | 10.6 | 80 | 19.08 | Translucent, flexible. |
| 2:1 | Methylcellulose 50 mPa. s | Propylene glycol | 13.0 | 92 | 24.96 | Translucent, hard and brittle. |
| 3:1 | Methylcellulose 50 mPa. s | Propylene glycol | 18.88 | 69 | 31.81 | Translucent, hard and brittle. |
| 1:1<br>3:1<br>4:1<br>6:1 | (a) Hydroxy propyl methylcellulose<br>(b) Methylcellulose | Triacetin | 20.0 | 25–50 | 25.30 | Transparent or translucent flexible. |
| 2:1<br>3:1<br>4:1 | Hydroxy propyl methylcellulose 15 mPa. s | Glycerine + triacetin | 20.0 | 50 | 30 | Translucent, flexible. |

* water insoluble polymer weight/water soluble polymer weight

From Table II, it is apparent that the film characteristics are substantially improved utilising annealing agents in conjunction with plasticizers. Further it was found that such films displayed desirable dosage coating characteristics with regard to dissolution in gastric liquids, water absorption, water barrier, properties and appearance. However, certain of the plasticizers, particularly glycerin, appear to evaporate in a matter of days. Triacetin plasticizers appear to be permanent.

Example 9

To 1 part of a 25.5 percent ethyl cellulose latex also containing therein two parts of a 5 percent methylcellulose annealing solution and between 25 to 50 percent triacetin plasticizer was added 20 percent (on a weight to weight basis of total polymer) of titanium dioxide*. Films were cast by evaporating the solvent at 30°C on a glass substrate. The addition of the titanium dioxide improved general film quality substantially in that the films were flexible and tougher than similar films without the opacifying agent. Also, the appearance and texture of the film were also improved. The addition of titanium dioxide to similar formulations utilizing glycerin in place of the triacetin decreased the "oily" surface appearance of the resulting films attributed to the presence of glycerin.

From the above results it becomes apparent that the latices of water insoluble polymers can be utilized to form excellent dosage coating compositions with the inclusion of plasticizers, annealing agents, particularly water soluble polymeric materials and various other essentially conventional constituents utilized generally with more conventional tablet coating compositions, such as opacifying agents, polishing agents, carnauba wax and chlorowax.

A particularly important quality of this invention will be appreciated with reference to Example 10.

Example 10

In the manner essentially as set forth in Example 2, a number of latices were prepared utilizing, as a fixed portion, ten parts of ethyl cellulose, 10 mPa.s (10 cps), 1 part cetyl alcohol, and 0.5 parts of sodium lauryl sulphate. Varying amounts of ethyl dichloride and water were employed, with the ethyl dichloride being present in the amount of 150 grams and water employed in the amount of 200 grams. Various solutions of between 20% and 30% by weight of ethyl cellulose relative to ethylene dichloride were prepared. After emulsification, the water and ethylene dichloride were removed from each sample until the resulting latex contained 34.5% ethyl cellulose in all instances. The viscosity of the ethyl cellulose in ethylene dichloride solutions increased rapidly

---

*25% plasticizer yields 16.67% solids loading and 50% yields 19.22% from the 10 Pa. s at 20% solutions, to 20 Pa. s at the 30% solution. Initially, the final viscosity of the latex after removing the organic solvent and some water to produce the 34.5% ethyl cellulose latex increased rapidly in viscosity, particularly at the 25% and the 27.5% solutions, but ultimately it was found that the 30% initial solution provided a final latex of relatively low viscosity, i.e., approaching that of the 20% initial solution and substantially below that of the 22.5% solutions. Investigation of the particle size disclosed that the 30% initial solution produced particles on the order of 5 to 10 μm in size which was substantially equivalent to the particle size and distribution of the 20% initial solution. This was surprising since the intermediate initial solutions, i.e. about 25% and 27.5%, produced less desirable latices having not only substantially greater particle size, i.e., up to 20 to 30 μm, but also a wider distribution of sizes. Thus it is preferred that the 30% initial solution be employed since the particle size is desirable and substantially less water need be removed to provide a latex of equivalent solids loading. Similar procedures may be employed to produce optimum initial solutions from other water insoluble polymers.

Example 11

Four petri dishes containing Difco Nutrient Agar were processed as described below and stored for three days at 37°C. One milliliter samples of ethyl cellulose and cellulose acetate phthalate latices, substantially as prepared in Examples 2 and 3, were spread over the surface of the agar prior to storage of two of the dishes. The samples of the cellulose latices were between 1 and 1½ years old and had been repeatedly opened during this period. However, no growth appeared on the agar, indicating that the samples were completely devoid of bacterial contamination. Finally, a control dish containing agar was exposed to the air prior to storage and also developed growth.

Thus, though the latices, according to the instant invention, are aqueous vehicle formulations, and though for all practical purposes the organic solvents have been removed, the latices still display a most surprising resistance to bacteria and mold growth. Though the precise reason for this quality of the instant invention is not entirely understood, it is believed to relate to the method of formation of the latex, i.e., initial dissolution in a water immiscible organic solvent, and then stripping the organic solvent to form the latex. Also, the smooth surface of the polymer particles in latex may not afford a forceable environment for growth of bacteria or molds. Systems employing cellulose polymers are preferred since thes are known to have a greater resistance to bacteria and mold growth and thus enhance the desirable characteristics of the instant invention. Certain additives, such as polyethylene glycol, preferably of a molecular weight 4,000 and 6,000, but operably between 400 and 20,000, may serve dual purposes. Such polyethylene glycols have been found to be desirable annealing agents while concurrently lending hing gloss to resulting tablet coatings. In most instances, polyethylene glycol would replace but a part of the annealing agents discussed above. However, worthwhile results are obtained when one or two parts of the polyethylene glycol is included on the basis of about 5 parts of ethyl cellulose in a typical formulation.

Example 12 – Enteric coated tablets

A latex solution was prepared by diluting 333.3 grams of ethylcellulose latex (30% solids) prepared according to example 1 and 19 grams of tributyl citrate with 147.7 grams of water to produce 500 grams of coating solution. 1.9 kg of blank lactones tablets were air suspension coated with this coating solution. Twenty four of the coated tablets were evaluated by the USP method for enteric coatings and the results set forth in Table III, arranged in order of increasing disintegration times in intestinal fluid.

### TABLE III
#### Disintegration time in minutes

| | | #1 | #2 | #3 | #4 | #5 | #6 |
|---|---|---|---|---|---|---|---|
| Round 1 | pH 1.2 | × | × | × | × | × | × |
| | pH 7.5 | a   b<br>17—37 | a   b<br>25—46 | a   b<br>29—49 | a   b<br>29—52 | a   b<br>36—58 | a   b<br>38—60 |
| Round 2 | pH 1.2 | × | × | × | × | × | × |
| | pH 7.5 | 28—47 | 32—48 | 32—56 | 35—56 | 40—61 | 41—60 |
| Round 3 | pH 1.2 | × | × | × | × | × | × |
| | pH 7.5 | 20—47 | 28—55 | 28—58 | 32—55 | 35—54 | 39—58 |
| Round 4 | pH 1.2 | × | × | × | × | × | × |
| | pH 7.5 | 29—51 | 29—48 | 33—51 | 34—58 | 36—57 | 41—65 |

Note:
x – no change in pH 1.2
a – start to disintegrate at pH 7.5
b – complete disintegration at pH 7.5

Example 13 – Resistance to bacteria

Ethylcellulose latices of this invention of 20 and 35% solids content (samples Nos. 1 and 2), prepared according to Example 1 were compared with commercially available coating compositions for resistance to bacteria. The pour-plate method of counting living bacteria or groups of bacteria was used. A measured or known amount of liquid latex was mixed with molten agar medium in a Petri-dish. After setting and incubation the number of colonies were counted. Counts were made on plates which were inoculated to yield between 30 and 300 colonies to ensure test precision and accuracy; the counts are recorded as the means of the most appropriate dilution.

Detailed description of the experiment

Using a fresh sterile pipette three dilutions of each latex sample (1, $^1/_{10}$, $^1/_{100}$) are prepared. One ml of each dilution is pipetted into each of 3 sterile tubes which contain 3 ml of molten agar at 45°C. The medium and inoculum are immediately mixed by shaking the tubes which are then poured to the ready made agar plate which contains approximately 15 ml of setting agar. Care is taken that the plate is kept flat on the bench during this operation. Each plate was then swirled in a circular movement lasting 5–10 seconds. After allowing the plates to set, they were inverted and incubated at room temperature for 4–5 days. After incubation a plate was selected containing between 30 and 300 colonies, the number of colonies are counted and the colony count per ml of initial latex sample is calculated from the dilution made for the preparation of the plate that was counted.

### TABLE IV
#### Viable bacteria colony count normalized to 0.1 ml latex samples following inoculation with E. Coli

| Sample | 0 h | 8 h | 1 day | 2 days |
|---|---|---|---|---|
| 1 | 15,400±529 | 0±0 | 0±0 | 0±0 |
| 2 | 16,000±200 | 0±0 | 0±0 | 0±0 |
| Water contaminated | 15,133±305 | 14,733±416 | 15,400±200 | 14,333±416 |

TABLE IV  (Fortsetzung)
Viable bacteria colony count normalized to 0.1 ml latex samples following inoculation with E. Coli

| Sample | 0 h | 8 h | 1 day | 2 days |
|---|---|---|---|---|
| A 4 mPa. s | 2,533±104 | 2,917±104 | 1,483±104 | 2,217±76 |
| A 13—18 mPa. s | 2,550±50 | 2,827±64 | 2,100±100 | 1,350±50 |
| A 40—60 mPa. s | 2,550±50 | 2,867±76 | 2,017±104 | 1,250±50 |
| B | 2,583±76 | 2,883±76 | 1,317±76 | 2,483±104 |
| Water contaminated | 2,600±50 | 2,833±76 | 2,317±76 | 1,533±104 |

Sample A Hydroxypropyl methylcellulose (Methocel (trade mark) from Dow Chemical Company, Midland, Michigan) E5 viscosity of 2% solution 2–6 mPa. s, E15 2% solution 13–18 mPa. s and E50 2% solution 40–60 mPa. s

Sample B Hydroxypropyl methylcellulose (Pharmacoat (trade mark), Shin-Etsu Chemical Company Ltd. Japan).

Example 14 – Non-enteric coated tablets

40 parts of the ethylcellulose latex, 30% solids, prepared by the method of example 1 was mixed with 50 parts of a 15% solids solution of HPMC, 5 mPa. s viscosity, and 10 parts of diethylphthalate. Blank tablets consisting of equal parts of microcrystalline cellulose and unmilled dicalcium phosphate dihydrate were ais suspension coated.

The resulting coated tablets were uniformly coated with a continuous film, which had a smooth glossy appearance. Twenty-four tablets were tested by the USP disintegration test and all passed.

In summary, the instant invention, while making use of components, constituents and individual procedures which per se have been heretofore employed in the formation of films, and occasionally for coating of tablets, provides unique results. For instance, while the water insoluble polymers employed in the instant invention may have heretofore been utilized in organosols for coating tablets, the instant invention provides for coating from latices. Many of the plasticizers, annealing agents, polishing agents, etc. have been heretofore employed with tablet coatings, but certainly not in conjunction with a latex of a water insoluble polymer which, in itself, is only marginally film forming. In no instance has there been any suggestion of a tablet coating composition utilizing an aqueous vehicle which is highly resistant to bacterial and mold growth. However, by combining the rather improbable pseudolatices, and more conventional additives, a dense, high gloss dosage coating may be formed which displays healing of initial coating imperfections upon application of subsequent layers. Such coatings display low water vapor transmission rates and are, depending upon the specific blend, suitable for gastric or enteric release. Of particular significance is the most surprising characteristic of bein sterile and resistant to bacterial growth and mold growth, though utilizing a water vehicle. High ratios of coating materials to water may be obtained, thereby greatly facilitating the formation of dosage coatings without unduly subjecting the dosages to attack by water, or requiring a great number of individual coatings with substantial amounts of solvent to be driven off. Further, given the disclosure of the instant invention, the basic mechanism, i.e., direct or inversion emulsification in a continuous water phase of a water insoluble polymer dissolved in an organic solvent, and formation of a pseudolatex therefrom with further addition of appropriate plasticizers, annealing agents, polishing agents and other such conventional additives to form a coating, may be readily varied and tailored for specific uses employing relatively conventional skills of the art. Thus, the advantages in many respects, and particularly the very unusual resistance to bactrial growth and mold growth, are intrinsic in the process and do not require deviations or compromises beyond the rather straigthforward formation of the pseudolatex.

**Claims**

1. A process for forming aqueous coatings on food or pharmaceutical dosage forms characterised by:
dissolving at least one substantially water insoluble polymer selected from ethyl cellulose, cellulose acetate phthalate, hydroxy propyl methyl cellulose phthalate and polyvinyl acetate phthalate in a volatile water immiscible organic solvent for the polymer:
forming a fine dispersion of the water insoluble polymer solution in a continuous aqueous phase by agitation of the water and the polymer solution, optionally in the presence of an emulsifying agent and/or an emulsion stabilizer;
forming solid particles of the water insoluble polymer in the water phase having an average particle size substantially between 0.1 and 20 μm by distillation of the organic solvent from the dispersion; adding a water soluble polymer and a plasticiser and optionally an additive to enhance the properties of the dispersion and/or improve the appearance of the coating, so as to obtain a latex having solids content from 16.67% to 60% by weight;
forming a film coating on a food or pharmaceutical dosage form by applying the latex to the dosage form and removing the water therefrom.

2. A process according to claim 1 characterised in that the aqueous dispersion includes a polishing agent.

3. A process according to claim 1 or claim 2 characterised in that the emulsion of organic solvent solution and water is formed with the benefit of an emulsifying agent.

4. A coated food or pharmaceutical dosage form produced according to any preceding claim.

5. A food or pharmaceutical dosage form coated with the dried residue of a latex which is an aqueous dispersion of at least one water insoluble polymer selected from ethyl cellulose, cellulose acetate phthalate, hydroxy propyl methyl cellulose phthalate and polyvinyl acetate phthalate having an average particle size between 0.1 and 20 μm, the solids content of the dispersion comprising from 16.67% to 60% by weight of the dispersion and containing an annealing agent in the form of a watersoluble polymer together with a plasticiser and optionally an emulsifier, or an additive to enhance the properties of the dispersion or improve the quality, performance or appearance of the product coating.

## Revendications

1. Procédé pour la formation de revêtements aqueux sur des formes d'administration alimentaires ou pharmaceutiques, caractérisé par:
la dissolution d'au moins un polymère essentiellement insoluble dans l'eau choisi parmi l'éthylcellulose, l'acétate-phtalate de cellulose, le pthalate d'hydroxypropylméthylcellulose et l'acétate-phthalate de polyvinyle dans un solvant organique volatil non miscible à l'eau du polymère;
la formation d'une dispersion fine de la solution de polymère insoluble dans l'eau dans une phase aqueuse continue par agitation de l'eau et de la solution de polymère, éventuellement en présence d'un agent émulsifiant et/ou d'un stabilisant d'émulsion;
la formation dans la phase aqueuse de particules solides du polymère insoluble dans l'eau ayant une taille moyenne des particules essentiellement entre 0,1 et 20 μm par élimination par distillation du solvant organique de la dispersion; l'addition d'un polymère soluble dans l'eau et d'un plastifiant et éventuellement d'un additif pour améliorer les propriétés de la dispersion et/ou pour améliorer l'aspect du revêtement de façon à obtenir un latex ayant une teneur en matières solides de 16,67 à 60% en poids;
la formation d'un revêtement pelliculaire sur une forme d'administration alimentaire ou pharmaceutique par application du latex à la forme d'administration et élimination de l'eau de celui-ci.

2. Procédé selon la revendication 1, caractérisé en ce que la dispersion aqueuse comprend un agent polissant.

3. Procédé selon la revendication 1 ou la revendication 2, caractérisé en ce que l'émulsion de la solution dans un solvant organique et d'eau est formée à l'aide d'un agent émulsifiant.

4. Forme d'administration alimentaire ou pharmaceutique revêtue produite selon l'une quelconque des revendications précédentes.

5. Forme d'administration alimentaire ou pharmaceutique revêtue du résidu séché d'un latex qui est une dispersion aqueuse d'au moins un polymère insoluble dans l'eau choisi parmi l'éthylcellulose, l'acétate-phthalate de cellulose, le pthalate d'hydroxypropylméthylcellulose et l'acétate-phthalate de polyvinyle ayant une taille moyenne des particules entre 0,1 et 20 μm, la teneur en matières solides de la dispersion étant comprise entre 16,67% et 60% du poids de la dispersion et contenant un agent de recuit sous forme d'un polymère soluble dans l'eau avec un plastifiant et éventuellement un émulsifiant ou un additif pour améliorer les propriétés de la dispersion ou améliorer la qualité, les performances ou l'aspect du revêtement.

## Patentsprüche

1. Verfahren zur Bildung wässriger Überzüge auf Nahrungsmitteln oder pharmazeutischen Dosierungsformen, dadurch gekennzeichnet, dass man mindestens ein im wesentlichen wasserunlösliches Polymer, ausgewählt unter Ethylcellulose, Celluloseacetatphthalat, Hydroxypropylmethylcellulosephthalat und Polyvinylacetatphtalat, in einem flüchtigen, mit Wasser nicht mischbaren organischen Lösungsmittel für das Polymer löst; eine Feindispersion der wasserunlöslichen Polymerlösung in einer kontinuierlichen wässrigen Phase durch Rühren des Wassers und der Polymerlösung gegebenenfalls in Gegenwart eines Emulgators und/oder eines Emulsionsstabilisators herstellt; feste Teilchen des wasserunlöslichen Polymers in der wässrigen Phase mit einer durchschnittlichen Teilchengrösse im wesentlichen zwischen 0,1 und 20 μm durch Destillation des organischen Lösungsmittels aus der Dispersion herstellt; ein wasserlösliches Polymer und einen Weichmacher sowie gegebenenfalls ein Additiv zur Förderung der Eigenschaften der Dispersion und/oder zur Verbesserung desAussehens des Überzuges zugibt, um einen Latex mit einem Feststoffgehalt von 16,67 bis 60 Gew.-% zu erhalten;
einen Filmüberzug auf einem Nahrungsmittel oder einer pharmazeutischen Dosierungsform durch Aufbringen des Latex auf die Dosierungsform und Entfernen des Wassers herstellt.

2. Verfahren nach Anspruch 1, dadurch gekennzeichnet, dass die wässrige Dispersion ein Poliermittel enthält.

3. Verfahren nach Anspruch 1 oder Anspruch 2, dadurch gekennzeichnet, dass die Emulsion aus organischer Lösungsmittellösung und Wasser mit Hilfe eines Emulgators hergestellt wird.

4. Überzogenes Nahrungsmittel oder pharmazeutische Dosierungsform, hergestellt nach irgendeinem der vorangehenden Ansprüche.

5. Nahrungsmittel oder pharmazeutische Dosierungsform, überzogen mit dem getrockneten Rückstand eines Latex, der eine wässrige Dispersion mindestens eines wasserunlöslichen Poly-

mers, ausgewählt unter Ethylcellulose, Cellulo-seacetatphthalat, Hydroxypropylmethylcellulo-sephthalat und Polyvinylacetatphthalat, mit einer durchschnittlichen Teilchengrösse zwischen 0,1 und 20 μm darstellt, wobei der Feststoffgehalt der Dispersion 16,67 bis 60 Gew.-% der Dispersion ausmacht und diese ein Haftmittel in Form eines wasserlöslichen Polymers zusammen mit einem Weichmacher und gegebenenfalls einem Emulgator oder einem Additiv zur Förderung der Eigenschaften der Dispersion oder Verbesserung der Qualität, der Gebrauchseigenschaften oder des Aussehens des Produktüberzuges enthält.